# EUROPÄISCHE PATENTSCHRIFT

(11) **EP 2 877 091 B1**
(45) Veröffentlichungstag und Bekanntmachung des Hinweises auf die Patenterteilung: **09.11.2016**
(21) Anmeldenummer: 13745002.9
(22) Anmeldetag: 22.07.2013
(51) Int. Cl.: A61B 5/103, A61B 5/00, A43B 3/00

(54) **VORRICHTUNG UND VERFAHREN ZUR LASTGRÖßENERFASSUNG AN EINEM BIOLOGISCHEN SYSTEM**
DEVICE AND METHOD FOR SENSING THE SIZE OF A LOAD ON A BIOLOGICAL SYSTEM
DISPOSITIF ET MÉTHODE DE DÉTECTION DE LA GRANDEUR D'UNE CHARGE APPLIQUÉE À UN SYSTÈME BIOLOGIQUE

(30) Priorität: 24.07.2012 DE 102012106715
(43) Veröffentlichungstag der Anmeldung: 03.06.2015
(73) Patentinhaber: Fachhochschule Münster, 48149 Münster (DE)
(72) Erfinder: STIEF, Thomas, 48145 Münster (DE); PEIKENKAMP, Klaus, 48161 Münster (DE)
(74) Vertreter: Viering, Jentschura & Partner mbB Patent- und Rechtsanwälte
(86) Internationale Anmeldenummer: PCT/EP2013/065438
(87) Internationale Veröffentlichungsnummer: WO 2014/016260

(56) Entgegenhaltungen:
- EP-A2- 1 709 904
- WO-A2-2006/083913
- DE-A1- 3 732 891
- DE-A1- 19 533 212

## Beschreibung

Die Erfindung richtet sich auf eine Vorrichtung zur Erfassung mindestens einer an einem Teil eines biologischen Systems auftretenden mechanischen Lastgröße, wobei die Vorrichtung eine an dem Teil, insbesondere einer Extremität, einem Gelenk oder einem Knochen, des biologischen Systems lastaufnehmend angeordnete oder anordnenbare, elastisch verformbare Trägerschicht aufweist, an welcher mindestens ein eine Messstelle oder ein Messfeld ausbildendes Messmittel angeordnet ist, das mindestens einen Kraft- und/oder Dehnungssensor aufweist.

Die Erfindung richtet sich weiterhin auf ein Verfahren zur Erfassung mindestens einer an einem Teil, insbesondere einer Extremität, einem Gelenk oder einem Knochen, eines biologischen Systems auftretenden mechanischen Lastgröße, wobei an dem Teil eine elastisch verformbare Trägerschicht lastaufnehmend angeordnet wird und wobei die Lastgröße an der elastisch verformbaren Trägerschicht mittels mindestens eines dort angebrachten Messmittels, insbesondere mittels eines Kraft- und/oder Dehnungssensors, ermittelt wird.

Schließlich richtet sich die Erfindung auf eine Verwendung einer vorstehend erwähnten Vorrichtung zur Durchführung eines erwähnten Verfahrens.

Unter einem biologischen System wird im Rahmen der vorliegenden Erfindung entsprechend der Systemtheorie nach Luhmann jedes Lebewesen verstanden, das sich durch seinen Körper von der Umwelt abgrenzt. Vereinfacht stellt jeder Körper eines Lebewesens somit ein biologisches System dar. Hierbei beschränkt sich der Begriff biologisches System aber nicht auf lebende Lebewesen, sondern kann auch tote Lebewesen umfassen. Teile eines solchen biologischen Systems sind dann insbesondere Extremitäten oder Gliedmaße oder Gelenke oder Knochen des Körpers eines Lebewesens. Hierbei ist es dann weiterhin so, dass auf dieses biologische System beispielsweise bei einer natürlichen Bewegung des lebenden Lebewesens auf ein solches "Teil" des biologischen Systems Kräfte einwirken oder eine Lastgröße einwirkt. Um die Erfassung derartiger Lastgrößen geht es im Wesentlichen bei der vorliegenden Erfindung. Hierbei ist die Erfassung einer Lastgröße aber nicht auf lebende Lebewesen oder lebende biologische Systeme beschränkt. Eine ganz wesentliche Ausrichtung ist aber die auf den Menschen, beziehungsweise den menschlichen Körper als ein biologisches System. Aber auch Tiere und hier insbesondere Säugetiere fallen unter den Begriff "biologisches System".

Fehlhaltungen und Beschwerden am menschlichen Muskel-Skelett-System treten bei vielen Menschen auf. Damit einhergehende Schmerzen können bei Erwerbstätigen zu einem vorzeitigen Ausscheiden aus dem Erwerbsleben führen.

Schuhe schützen und unterstützen die Füße in mechanischer Hinsicht, im dem sie beispielsweise auftretende Drücke dämpfen, den Fuß führen und stützen.

Bestehen Fehlhaltungen und Beschwerden, ist das Wiederherstellen der richtigen Funktionalität oder eine Entlastung der Füße über eine Modifikation der Schuhe und der Sohlen ein entscheidender Faktor in der orthopädischen Versorgungspraxis. Um dies zu gewährleisten, ist die Ermittlung der während des Stehens, Gehens und Laufens in den Schuhen und an den Füßen auftretenden, mehraxialen mechanischen Beanspruchungen und Belastungen von großem Interesse. Dies gilt insbesondere bei einer Überprüfung von orthopädischen Veränderungen an einem Schuh, einer Prothese oder einer Schuhsohle, die eine Verbesserung der Qualität der Bewegung bewirken sollen.

In der Praxis der Biomechanik, der Sportwissenschaft, der Schuhindustrie und der Technischen Orthopädie ist es bisher üblich, im Schuh auftretende Belastungen mittels plantarer Druckmessung unter Zurhilfenahme entsprechender Innensohlensysteme zu erfassen und zu ermitteln.

Mit derartigen Messsystemen können aber nur Kräfte und deren Wirkung analysiert werden, die senkrecht zur Sohle auftreten. Es können also lediglich einaxiale Beanspruchungen festgestellt werden.

Die Betrachtung mehraxialer Spannungszustände bei der Schuh- und Fußbeanspruchung sind mit derartigen Vorrichtungen nicht möglich. Entlastende Maßnahmen, zum Beispiel des Vorfußbereichs, welche bei diversen orthopädischen und systemischen Erkrankungen indiziert sind, lassen sich auf diese Weise daher nur unzureichend überprüfen. Das liegt u.a. daran, dass mit diesen Systemen nur Normalkräfte oder deren Veränderung, beispielsweise infolge einer Veränderung des Sohlenaufbaus, gemessen werden können.

Eine gattungsgemäße Vorrichtung zur Messwerterfassung und - auswertung in Schuhen oder Schuheinlagen ist aus der DE 102 01 134 A1 bekannt. Diese Vorrichtung offenbart ein Messsystem, mit welchem auch Torsionsbelastungen sowie Biege-, Schub-, Torsions- und Längsspannungen an den Füßen erfasst werden können. Basis hierfür ist eine mit Messfeldern ausgestattete Schuheinlegesohle, wobei die gewünschten Informationen in den Messfeldern mit piezoelektrischen Effekten oder Elementen, aber auch mit Dehnungsmessstreifen, erfasst und die jeweils erzeugten Spannungssignale verarbeitet werden können. Allerdings ist diesem Dokument nicht die genaue Anordnung und Positionierung der eigentlichen Messwertaufnehmer zu entnehmen.

Eine gattungsgemäße Vorrichtung offenbaren auch die DE 198 10 182 C1 und die EP 1 709 904 A2.

Eine auf Druckmesssensoren basierende Vorrichtung ist zudem aus der DE 10 2010 049 154 A1 bekannt. Diese Druckschrift offenbart eine Sohle mit einem Sensor für den Einsatz in einem Schuh, wobei die mechanische Druckverteilung an der menschlichen Fußsohle mit Sensoren und einer Elektronik gemessen wird. Dabei sind die Sensoren und die Elektronik auf einer Platine angeordnet. Als Sensoren werden kapazitive Drucksensoren eingesetzt.

Der Erfindung liegt die Aufgabe zugrunde, eine gegenüber dem Stand der Technik verbesserte Lösung zu schaffen, die die Durchführung einer mehraxialen Spannungsanalyse von an einer lastaufnehmenden Vorrichtung angreifenden Kräften oder Belastungen, die von einem Teil eines biologischen Systems ausgeübt werden, ermöglicht.

Nach einem Aspekt der Erfindung soll insbesondere die Durchführung einer mehraxialen Spannungsanalyse von an einer in oder an einem Schuh angeordneten Schuhsohle beim Tragen des Schuhs auftretenden dynamischen und statischen Belastungen ermöglicht werden, ohne dass hierbei eine Bodenreaktionskraft einwirken muss.

Bei einer Vorrichtung der eingangs näher bezeichneten Art wird diese Aufgabe erfindungsgemäß dadurch gelöst, dass die mindestens eine Messstelle oder das mindeste eine Messfeld mindestens zwei an der Trägerschicht einander gegenüberliegend angeordnete Messmittel, insbesondere Kraft- und/oder Dehnungssensoren, aufweist.

Ebenso wird diese Aufgabe erfindungsgemäß bei einem Verfahren der eingangs näher bezeichneten Art dadurch gelöst, dass die mechanische Lastgröße mittels mindestens zweier, an einer Messstelle oder einem Messfeld einander an der Trägerschicht gegenüberliegend angeordneter Messmittel, insbesondere Kraft- und/oder Dehnungssensoren, erfasst wird.

Schließlich wird diese Aufgabe erfindungsgemäß auch durch die Verwendung einer Vorrichtung nach einem der Ansprüche 1 - 12 zur Durchführung eines Verfahrens nach einem der Ansprüche 13 - 15 gelöst.

Vorteilhafte Weiterbildungen und zweckmäßige Ausgestaltungen der Erfindung sind Gegenstand der jeweiligen abhängigen Ansprüche.

Mit der Erfindung ist es möglich, an einer Messstelle oder in einem Messfeld mehraxiale Spannungen oder Dehnungen der Trägerschicht, insbesondere der Schuhsohle, während einer Bewegung des biologischen Systems und hier des messtechnisch erfassten Teils des biologischen Systems, insbesondere des menschlichen Fußes, zu detektieren. Hierzu umfasst die Vorrichtung wenigstens eine elastisch verformbare Trägerschicht, insbesondere Messsohle, mit wenigstens einer Messstelle oder einem Messfeld. Die Messstelle weist an gegenüberliegenden Seiten - vorzugsweise oben und unten - jeweils ein Messmittel auf. Diese Anordnung der Messmittel ist vorteilhaft, weil sie einen Vergleich und eine kombinatorische Verarbeitung der auf einer Oberseite oder ersten Seite und einer Unterseite oder zweiten Seite derselben Messstelle gemessenen, d.h. erfassten, Werte erlaubt. Eine derartige Anordnung von Messmitteln an mehreren verschiedenen Messstellen oder Messfeldern der Trägerschicht, insbesondere Schuhsohle, ermöglicht es, einen Überblick über die Belastung der Trägerschicht, insbesondere Schuhsohle, und somit des auf der Trägerschicht aufliegenden oder an dieser anliegenden Teils des biologischen Systems, insbesondere des auf der Schuhsohle aufliegenden Fußes, zu erhalten und eine mehraxiale Spannungsanalyse durchzuführen.

In Ausgestaltung sieht die Erfindung daher vor, dass das Teil ein menschlicher Fuß ist. Hierbei ist es besonders zweckmäßig, dass die Trägerschicht Bestandteil einer Schuhsohle ist oder eine solche ausbildet, oder dass die Trägerschicht Bestandteil einer Schuhsohle ist oder eine solche ausbildet, wobei die Schuhsohle als herausnehmbare Einlegesohle ausgebildet ist, was die Erfindung ebenfalls vorsieht.

In weiterer Ausgestaltung sieht die Erfindung vor, dass mindestens eines der Messmittel einen mehrachsigen Spannungs- und/oder Dehnungszustand, insbesondere eine elastische Verformung, der Trägerschicht, insbesondere Schuhsohle, detektierender Kraft- und/oder Dehnungssensor ist.

Hierbei ist es dann weiterhin zweckmäßig, wenn mindestens einer der Kraft- und/oder Dehnungssensoren ein einen statischen und/oder dynamischen mehrachsigen Spannungs- und/oder Dehnungszustand der Trägerschicht, insbesondere Schuhsohle, detektierender Kraft- und/oder Dehnungssensor ist. Insbesondere mit mehrachsige, insbesondere zweiachsige, Spannungszustände detektierenden Kraft- und/oder Dehnungssensoren lassen sich in vorteilhafter Weise Innensohlensysteme für Schuhe bereitstellen, mit welchen dynamische mehraxiale Spannungen oder Dehnungen der Schuhsohle während der menschlichen Bewegung im Schuh detektiert werden können.

Hierbei kann mindestens einer der Kraft- und/oder Dehnungssensoren ein Biegemomente und/oder Torsionsmomente und/oder Biegespannungen und/oder Torsionsspannungen der Trägerschicht, insbesondere Schuhsohle, detektierender Kraft- und/oder Dehnungssensor sein. Insbesondere aber sind gemäß Ausgestaltung der Erfindung die jeweils mindestens zwei an einer Messstelle oder einem Messfeld einander gegenüberliegend angeordneten Messmittel, insbesondere Kraft- und/oder Dehnungssensoren, einen mehrachsigen Spannungs- und/oder Dehnungszustand, insbesondere eine elastische Verformung, der Trägerschicht, insbesondere Schuhsohle, detektierende Kraft- und/oder Dehnungssensoren.

Insbesondere durch die Kombination von mindestens zwei an einer Messstelle oder in einem Messfeld gegenüberliegend zueinander angeordneten Kraft- und/oder Dehnungssensoren mit deren Ausbildung als einen mehrachsigen Spannungs- und/oder Dehnungszustand und detektierende Kraft- und/oder Dehnungssensoren lassen sich die gewünschten dynamischen oder statischen mehraxialen Spannungsanalysen und mehraxialen Dehnungen der Trägerschicht, insbesondere Schuhsohle, während der Bewegung des jeweils untersuchten Teils des biologischen Systems, insbesondere des menschlichen Fußes in einem Schuh, detektieren.

Als eine besonders vorteilhaft zu realisierenden Ausführung sieht die Erfindung weiterhin vor, dass eine Messstelle oder ein Messfeld eine Trägerschicht, insbesondere Schuhsohle, mit einer ersten Oberfläche und der zweiten Oberfläche aufweist, wobei je Messstelle oder je Messfeld jeweils auf der ersten Oberfläche und der zweiten Oberfläche der Trägerschicht, insbesondere Schuhsohle, mindestens ein einen mehrachsigen Spannungs- und/oder Dehnungszustand der Trägerschicht, insbesondere Schuhsohle, detektierender Kraft- und/oder Dehnungssensor angeordnet ist.

Eine besonders zweckmäßige Ausgestaltung der Schuhsohle besteht darin, dass die Schuhsohle als herausnehmbare Einlegesohle ausgebildet ist.

Die Schuhsohle oder die Trägerschicht besteht vorzugsweise aus einem Material, insbesondere Edelstahl, das beispielsweise in einer Schuhsohle angeordnet oder als eine solche ausgebildet bei einer natürlichen Belastung durch einen menschlichen Fuß ausschließlich in seinem elastischen Bereich beansprucht, insbesondere verformt, wird.

Der mindestens eine Kraft- und/oder Dehnungssensor, vorzugsweise alle Kraft- und/oder Dehnungssensoren, kann/können als Dehnungsmessstreifen (DMS) ausgebildet sein, was die Erfindung ebenfalls vorsieht. Zweckmäßig kann es dann weiterhin sein, wenn der Dehnungsmessstreifen (DMS) und/oder die Dehnungsmessstreifen (DMS) jeweils als DMS-Rosette, insbesondere als 90°-DMS-Rosette, ausgebildet ist/sind. Eine besonders vorteilhafte Weiterbildung besteht darin, dass sich jeweils gegenüberliegend angeordnete Kraft- und/oder Dehnungssensoren, insbesondere Dehnungsmessstreifen (DMS), elektrisch in Form einer Wheatstone'schen Brückenschaltung, insbesondere als Halbbrücke, miteinander verschaltet sind. Mit einer solchen speziellen elektrischen Verschaltung jeder der Messstellen lässt sich problemlos ein Vergleich und eine kombinatorische Verarbeitung der jeweils gegenüberliegend auf einer Oberseite und deiner Unterseite derselben Messstelle oder desselben Messfelds gemessenen, d.h. erfassten, Werte, d.h. der jeweils ausgelösten elektrischen Signale, durchführen.

In dieser Ausgestaltung zeichnet sich die Vorrichtung in Abgrenzung gegenüber den bisher bekannten Vorrichtungen und Verfahren dadurch aus, dass aufgrund der Anordnung von mindestens je einem Messmittel pro Messstelle oder Messfeld, beispielsweise auf der Oberseite und der Unterseite einer Schuhsohle oder Messsohle, und aufgrund der speziellen Verschaltung (Wheatstone'sche Brücke) der Messmittel eine eindeutige Zuordnung der Belastungsform möglich ist (z.B. eine eindeutige Unterscheidung zwischen Biege- und Zugbelastung).

In einer besonders vorteilhaften Ausgestaltung kann dann ferner vorgesehen sein, dass der mindestens eine Kraft- und/oder Dehnungssensor kraftschlüssig mit der Schuhsohle und/oder der Trägerschicht verbunden ist, wobei vorzugsweise der mindestens eine Kraft- und/oder Dehnungssensor oder die Kraft- und/oder Dehnungssensoren und die Trägerschicht von einer hochelastischen Materialschicht überdeckt sind.

Bei einer kraftschlüssigen Verbindung eines Dehnungsmessstreifens (DMS) mit der Trägerschicht können während des Gehens auftretende Krümmungen der Trägerschicht gut mit dem DMS gemessen werden. Weiterhin kann hierbei durch einen Vergleich der Messwerte an einer Ober- und einer Unterseite der Messstelle oder des Messfeldes bestimmt werden, ob eine Biegung der Schuhsohle vorliegt, und in welche Richtung die Sohle beim jeweiligen Belastungszustand gebogen wird.

Sofern die Schuhsohle mehrere Messstellen mit jeweils einem oben und unten oder auf gegenüberliegenden Seiten der Trägerschicht angeordneten Dehnungsmessstreifen (DMS) aufweist, ist es auch möglich, festzustellen, ob und wo die Trägerschicht verdreht wird.

Dehnungsmessstreifen sind kostengünstig zu beschaffen und ermöglichen eine präzise Messung der auftretenden Spannungen an den gegenüberliegenden Oberflächen der Trägerschicht bzw. an der Ober - und Unterseite der auf der Sohle definierten Messstellen und Messfelder.

Weiterhin ist möglich, Anzahl und Ort der Messstellen und Messfelder abhängig vom vorliegenden Messproblem, auszuwählen, was durch das jeweilige biologische System, das zu untersuchende Teil des biologischen Systems, beispielsweise die Schuhgröße, die Sohlengröße, die Sohlenform, aber auch die Belastung und/oder die gewünschte Auflösung der Messergebnisse beeinflusst wird. Dabei behindert die Anzahl und die erfindungsgemäße Anordnung der Messmittel die Bewegung des Teils, insbesondere des Fußes, nicht, was vorteilhaft ist.

Wenn vorgesehen ist, dass der oder die Dehnungsmessstreifen als DMS-Rosette(n) ausgeführt ist/sind, ist eine 90°-DMS-Rosette, die auch als "T"-Rosette bezeichnet wird, vorteilhaft. Eine DMS-Rosette hat die Aufgabe, die Längenänderungen an der Messstelle zu ermitteln, erfasst dabei aber einen zweiachsigen, also mehrachsigen, Spannungszustand.

90°-Rosetten werden bevorzugt eingesetzt, wenn die Hauptdehnungsrichtungen oder Hauptspannungsrichtungen aufgrund vorhergehender Überlegungen bekannt sind. Sind die Hauptdehnungsrichtungen oder Hauptspannungsrichtungen nicht bekannt, kommen bevorzugt 3-Element-Rosetten zum Einsatz wie 45°-, Rechtwinkel- oder 60°-Delta-Rosetten.

Abhängig von der Größe und der Form der Trägerschicht und der Anzahl der Messstellen können planar oder gekreuzt ausgeführte Rosetten zur Messung eingesetzt werden. Planare Rosettenausführungen decken einen flächenmäßig größeren Bereich ab als gekreuzte Rosettenausführungen.

Insbesondere weisen einander gegenüberliegend angeordnete 90°-DMS-Rosetten eine Wheatstone'sche Brückenschaltung auf.

Diese Schaltungstechnik ermöglicht aber auch bei allen anderen erfindungsgemäßen Anordnungen der Kraft- und/oder Dehnungssensoren in Form von Dehnungsmessstreifen eine klare Abgrenzung einer in den einzelnen, jeweils einem Messfeld zugeordneten Bereichen eines auf der Trägerschicht anliegenden Teils eines biologischen Systems, beispielsweise eines auf der Sohle aufliegenden Fußes, auftretenden, mehraxialen Sohlenbelastung und Beanspruchung.

Die Trägerschicht oder die Schuhsohle ist elastisch und weist definierte bekannte mechanische Materialeigenschaften, wie beispielsweise ein bekanntes Elastizitätsmodul, auf. Die während einer Bewegung des Fußes auftretenden Krümmungen des untersuchten Teils, beispielsweise des Fußbettes, führt die Trägerschicht oder die Schuhsohle mit aus. Für die dabei auftretenden Formänderungen der Trägerschicht oder Schuhsohle werden die Biege- und Torsionsspannungen mittels der jeweiligen Kraft- und/oder Dehnungssensoren, beispielsweise mittels 90°-DMS-Rosetten, erfasst.

Mit Hilfe des für das Trägerschichtmaterial oder Sohlenmaterial bekannten Elastizitätsmoduls kann dann aus den erfassten Messwerten die an den Messstellen und in den Messfeldern auftretende Spannung berechnet werden, d.h. eine mehraxiale Spannungsanalyse durchgeführt werden.

In weiterer Ausgestaltung der erfindungsgemäßen Vorrichtung kann auch vorgesehen sein, dass die Vorrichtung einen Schuh umfasst, der eine Grund- oder Laufsohle und eine auf der Grund- oder Laufsohle angeordnete Schuhsohle, die die Messstellen oder die Messfelder aufweist, umfasst. Hierbei sieht die Erfindung dann insbesondere vor, dass die Schuhsohle eine erste elastomere Schutzschicht und eine darunter angeordnete erste Messschicht auf einer ersten Seite der Trägerschicht sowie eine auf einer zweiten Seite der Trägerschicht angeordnete zweite Messschicht und eine die zweite Messschicht schützend überdeckende zweite elastomere Schutzschicht aufweist.

Bei einer solchen Platzierung der Schuhsohle in einem Schuh liegt die Schuhsohle auf der Grund- oder Laufsohle im Schuh auf. Dabei weist die Grund- oder Laufsohle ein äußeres Grund- oder Laufprofil auf. Der Kontakt des Schuhs zu einer Unterlage wird durch die Grund- oder Laufsohle hergestellt. Schuhsohle und Grund- oder Laufsohle zusammen bilden den Sohlenbereich des Schuhs aus.

Die Schuhsohle, oder genauso jede erfindungsgemäße Trägerschicht, weist zweckmäßigerweise eine obere, erste elastomere Schutzschicht auf, eine darunter angeordnete erste Messschicht auf einer ersten (Ober)Seite der Trägerschicht, eine auf einer zweiten (Unter)Seite der Trägerschicht angeordnete zweite Messschicht und eine die zweite Messschicht schützende untere, zweite elastomere Schutzschicht auf. Die Messschichten weisen an definierten Positionen, den Messstellen, Messmittel auf. Die Messmittel sind dabei so angeordnet, dass sie sich an einer Messstelle oder einem Messfeld einander gegenüberliegen.

Die Kraft- und/oder Dehnungssensoren und die zugehörigen Mess- und Versorgungsleitungen können mit Silikon und die Trägerschicht mit flexiblem Ethylenvinylacetat (EVA; 25- 35 SH) abgedeckt werden. Diese Konstruktion ist vorteilhaft, weil so die Kraft- und/oder Dehnungssensoren, die Mess- und Versorgungsleitungen und die Schuhsohle einen mechanischen Schutz erhalten.

Für die Durchführung einer Auswertung der erhaltenen und erfassten Messsignale ist es zudem vorteilhaft, wenn dem mindestens einen Messmittel eine Auswerteeinheit zugeordnet ist, in der mittels in Kraft- und/oder Dehnungssensoren erzeugter elektrischer Spannungssignale und mittels in der Auswerteeinheit hinterlegter Materialkenngrößen, insbesondere Elastizitätsmodulen und Widerstandsmomenten, ein mehraxialer der Spannungs- und/oder Dehnungszustand an der jeweiligen Messstelle oder dem jeweiligen Messfeld ermittelt wird.

Hierbei können die Messmittel, insbesondere die Kraft- und/oder Dehnungssensoren, über Mess- und/oder Versorgungsleitungen mit einer Auswerteeinheit, einer Speichervorrichtung, einer Vorrichtung zur Energieversorgung und/oder einer Datenausleseeinheit elektrisch leitend verbunden sein.

Die Messmittel der erfindungsgemäßen Vorrichtung sind also gewünschtenfalls über die Mess- und Versorgungsleitungen mit einer Auswertungsvorrichtung, einer Speichervorrichtung, einer Vorrichtung zur Energieversorgung und einer Datenausleseeinheit elektrisch verbunden. Hierbei weist die Auswertevorrichtung vorzugsweise die spezielle elektrische Verschaltung in Form einer oder mehrerer Wheatstone'scher Brücken auf, wobei letztere aber auch in einer zur Auswertevorrichtung separaten Verschaltungseinrichtung oder Verschaltungsvorrichtung angeordnet sein kann/können. Die Messdaten können mittels Bluetooth ausgelesen werden. Eine andere Möglichkeit bietet beispielsweise eine Infrarotschnittstelle. Diese Auslesemöglichkeit - Bluetooth oder Infrarotschnittstelle - ist beispielsweise bei einer Überwachung von Sportlern während eines Wettkampfes vorteilhaft einsetzbar.

Das erfindungsgemäße Verfahren kann in Weiterbildung dadurch gekennzeichnet sein, dass als mechanische Lastgröße ein mehrachsiger Spannungs- und/oder Dehnungszustand, insbesondere eine elastische Verformung, der Trägerschicht erfasst wird. Hierbei ist es dann besonders zweckmäßig, wenn die mindestens eine mechanische Lastgröße mittels einer der Vorrichtungen nach einem der Ansprüche 1 - 12 erfasst und/oder ermittelt wird.

Von besonderem Vorteil ist das erfindungsgemäße Verfahren am menschlichen Körper als biologischem System anwendbar. Die mechanische Lastgröße kann an einem menschlichen Fuß erfasst werden. Hierbei ist es dann zweckmäßig, dass an dem Teil, insbesondere dem menschlichen Fuß, eine als Schuhsohle ausgebildete Trägerschicht angeordnet wird.

Die Erfindung ist nachstehend anhand einer Zeichnung beispielhaft näher erläutert. Diese zeigt in
- Fig. 1-5: in schematischer Darstellung ein mechanisches Fußmodell während des Gangzyklus der Schwungvorbereitung,
- Fig. 6: in schematischer Schnittdarstellung einen Sohlenaufbau eines Schuhs,
- Fig. 7a, 7b: in schematischer Schnittdarstellung eine Draufsicht von oben und von unten auf eine Schuhsohle,
- Fig. 8: eine schematische Darstellung einer erfindungsgemäßen Vorrichtung,
- Fig. 9: gemittelte Dehnungsverläufe in Längsrichtung einer Schuhsohle hinter dem Großzehengrundgelenk eines Fußes während des Gehens und in
- Fig. 10a-10e: schematisch das der Erfindung zugrundeliegende Messprinzip.

Nachstehend ist ein Ausführungsbeispiel der vorliegenden Erfindung näher dargestellt, wobei das biologische System (im Sinne der Systemtheorie von Luhmann) der menschliche Körper ist und das Teil des biologischen Systems, an welchem eine auftretende mechanische Lastgröße ermittelt werden soll, ein menschlicher Fuß 1 ist. Die erfindungsgemäß vorgesehene Trägerschicht 24 ist folglich Bestandteil einer Schuhsohle 3 bzw. bildet eine Schuhsohle 3 aus.

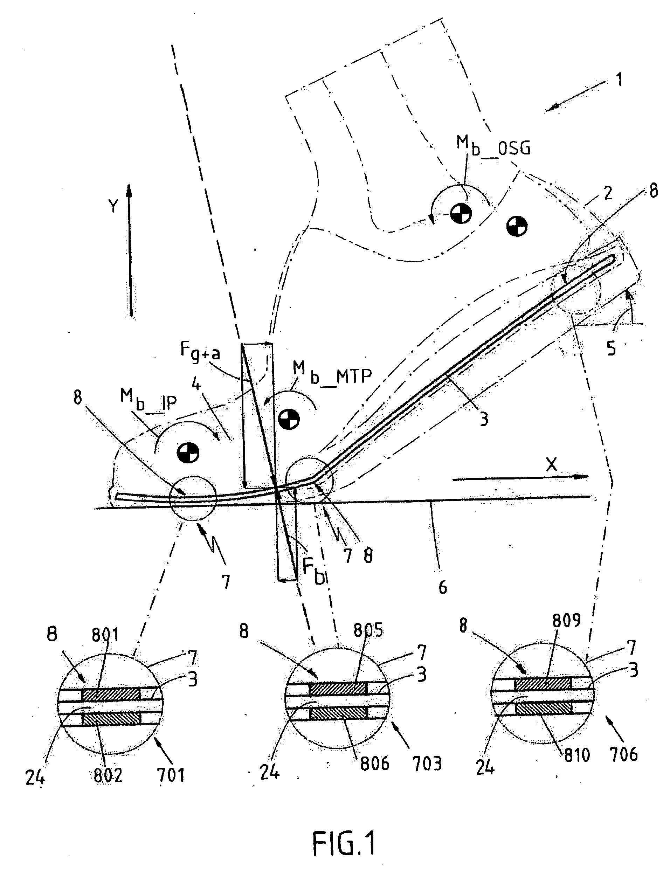

In der Figur 1 ist ein Fußmodell mit Schuh 2 während eines Gangzyklus in der Schwungvorbereitung dargestellt. Der Fuß 1 ist dabei von dem Schuh 2 umgeben. Der Fuß 1 liegt auf der Schuhsohle 3 auf. Die Belastung des Fußes 1 wird in dieser Fußstellung vom Vorfuß 4 aufgenommen. Der hintere Teil des Fußes 1 ist um einen Winkel 5 gegenüber einer Unterlage 6, auf welcher der Schuh 2 aufliegt, angehoben. Während dieses Zustandes, der als Momentaufnahme betrachtet werden kann, treten Schubkräfte parallel zur eingezeichneten x - Achse auf. Ursache der Schubkräfte ist das Abrollen des Vorfußes 4 und damit des Schuhs 2 auf der Unterlage 6. Diese Schubspannungen lassen sich mit der erfindungsgemäßen Vorrichtung erfassen.

In der Figur 1 sind mit F_{g+a}, der Gewichts- und Beschleunigungskraft des Körperschwerpunktes, und F_{b}, der Bodenreaktionskraft, zwei der bei dem Abrollvorgang des Vorfußes 4 auf der Unterlage 6 auftretenden Kräfte dargestellt. Beide Kräfte sind auf einer Wirkungslinie eingezeichnet. Sowohl F_{g+a} als auch F_{b} weisen Komponenten parallel zur x- und zu einer dazu senkrechten y- Achse auf.

Aufgrund der parallel zur x- Achse verlaufenden Kraftkomponenten treten Schubspannungen in der Schuhsohle 3 auf. Die parallel zur y- Achse verlaufenden Kraftkomponenten bewirken das Auftreten von Normalkräften und Normalspannungen in der Schuhsohle 3.

Zusätzlich sind drei Drehmomente M_{b_OSG}, das Biegemoment am oberen Sprunggelenk, M_{b_MTP}, das Biegemoment am Großzehengrundgelenk, und M_{b_IP}, das Biegemoment am Großzehenendgelenk, eingezeichnet. Diese Drehmomente sind vor allem für die Beanspruchung des Großzehengrundgelenks wesentlich.

Die Schuhsohle 3 weist mindestens eine Messstelle oder ein allgemein mit 7 bezeichnetes Messfeld auf. In der dargestellten Ausführungsform weist die Schuhsohle 3 sechs Messfelder 701, 702, 703, 704, 705, 706 auf, wovon in der Figur 1 die Messfelder 701, 703 und 706 erkennbar sind. Jedes der Messfelder 701 - 706 umfasst jeweils ein oberhalb und ein unterhalb einer Trägerschicht 24 angeordnetes Messmittel 801, 802, 803, 804, 805, 806, 807, 808, 809, 810, 811, 812 , wovon in der Figur 1 die dem Messfeld 701 zugeordneten Messmittel 801 und 802, die dem Messfeld 703 zugeordneten Messmittel 805 und 806 und die dem Messfeld 706 zugeordneten Messmittel 809 und 810 ersichtlich sind. Hierbei bildet zudem jedes der Messmittel 801 - 812 eine Messstelle aus.

Die einzelnen Messmittel 801 bis 812 sind jeweils als ein Kraft- und/oder Dehnungssensor 8 in Form eines Dehnungsmessstreifen (DMS) oder in Form mehrerer zusammenwirkender oder zusammengeschalteter Dehnungsmessstreifen (DMS) ausgebildet. Aufgabe der DMS ist es, jeweils Werte für eine Längenänderung oder einem mehrachsigen Spannungszustand im jeweiligen Messfeld zur Verfügung zu stellen. Über die erfassten Längenänderungen oder mehrachsigen Spannungszustände der DMS werden die je Messmittel 801 - 812 oder je Messstelle oder je Messfeld 7 an der Schuhsohle 3 auftretenden mehraxialen Spannungen oder Dehnungen als zu ermittelnde Lastgröße bestimmt. Hierzu sind die Messmittel 801 - 812 sich jeweils paarweise gegenüberliegend auf der Ober- und Unterseite einer Trägerschicht 24 angeordnet

Bevorzugt finden als Messvorrichtung 90°-DMS-Rosetten, die auch als "T"-Rosetten bezeichnet werden, Verwendung. Der Einsatz dieses Rosettentyps ist vorteilhaft, wenn die Hauptdehnungs- oder Hauptspannungsrichtungen der Schuhsohle 3 aufgrund vorhergehender Überlegungen bekannt sind. Sind die Hauptdehnungs- oder Hauptspannungsrichtungen unbekannt, kommen bevorzugt DMS in der Form von 3-Element-Rosetten zum Einsatz, gleich, ob als 45°-Rosette, Rechtwinkel-Rosette oder 60°-Delta-Rosette.

Die elektrische Verschaltung jeweils zweier sich an einem der Messfelder 701 -706 gegenüberliegender Kraft- und/oder Dehnungssensoren 8, vorzugsweise DMS-Rosetten, erfolgt jeweils mittels einer Wheatstone`schen Brückenschaltung, vorzugsweise einer elektrischen Halbbrücke. So lassen sich verschiedene, insbesondere mehraxiale, Spannungs- und Dehnungszustände mittels der grundsätzlich bekannten Verschaltungstechnik einer oder mehrere Wheatstone'schen Brücke(n) ermitteln und unterscheiden. Insbesondere ist dadurch eine klare Abgrenzung der an den verschiedenen Messfeldern 7 jeweils auftretenden mehrachsigen Schuhsohlenbelastungen und -beanspruchungen gegeneinander und deren eindeutige Identifizierung möglich. In dem Ausführungsbeispiel sind die Messfelder 701 und 702 im Vorfußbereich, die Messfelder 703 und 704 im Mittelfußbereich und die Messfelder 705 und 706 im Rückfußbereich angeordnet, wobei die Messfelder 701, 703 und 705 medial und die Messfelder 702, 704 und 706 lateral angeordnet sind.

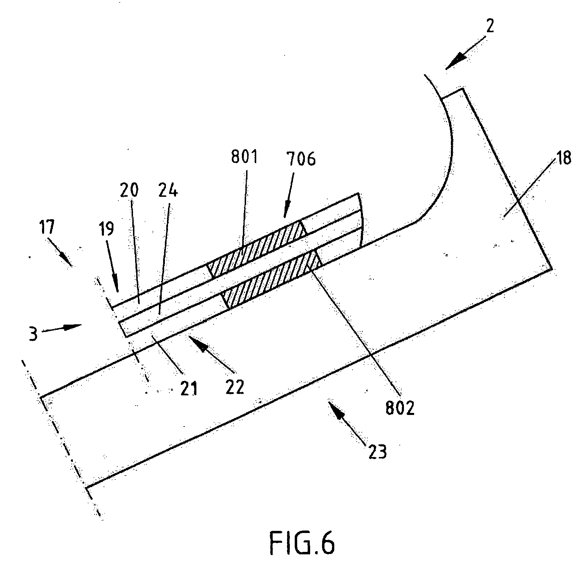

Der Einsatz einer Wheatstone'schen Messbrücke ist vorteilhaft, da so auch kleine Veränderungen in den Kraft- und/oder Dehnungssensoren 8 und damit in der Schuhsohle 3 gemessen werden können. Die Materialien und die Materialeigenschaften der Schuhsohle 3 und hier insbesondere die Materialeigenschaften einer Trägerschicht 24 (siehe Fig. 6) sind so ausgewählt, dass die Schuhsohle 3 und/oder die Trägerschicht 24 durch die auftretenden Belastungen und Lastgrößen ausschließlich im elastischen Bereich verformt werden. Über bekannte Materialkennwerte wie Elastizitätsmodul und Widerstandsmomente lassen sich mittels der Messmittel und einer geeigneten zugeordneten Auswerteeinheit dann die gewünschten Lastgrößen, Beanspruchungen, und mehrachsigen Spannungszustände der Schuhsohle 3 und die an den Messfeldern 701 bis 706 wirkenden Momente ermitteln. Es sind die Ermittlung charakteristischer und relevanter Größen der Fuß- und Schuhbeanspruchung, die mehraxiale Betrachtung der Fuß- und Schuhbeanspruchung und eine Beanspruchungsanalyse ohne Wirkung der Bodenreaktionskraft möglich.

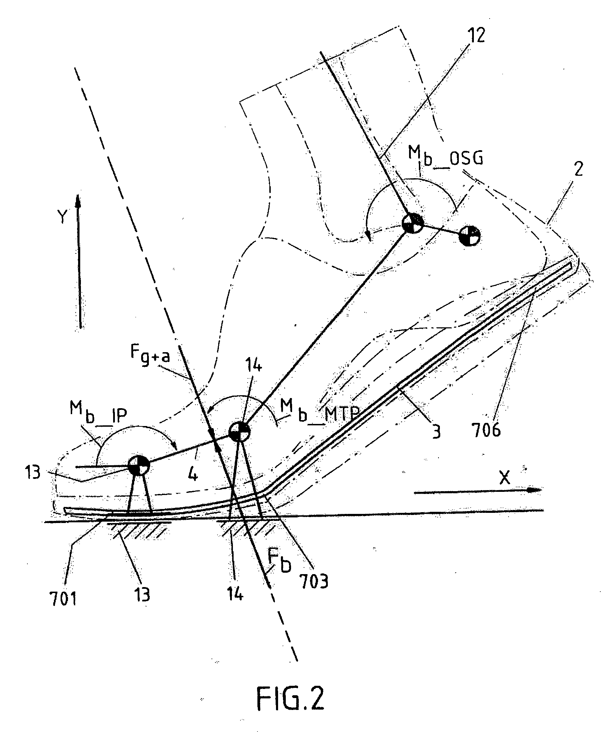

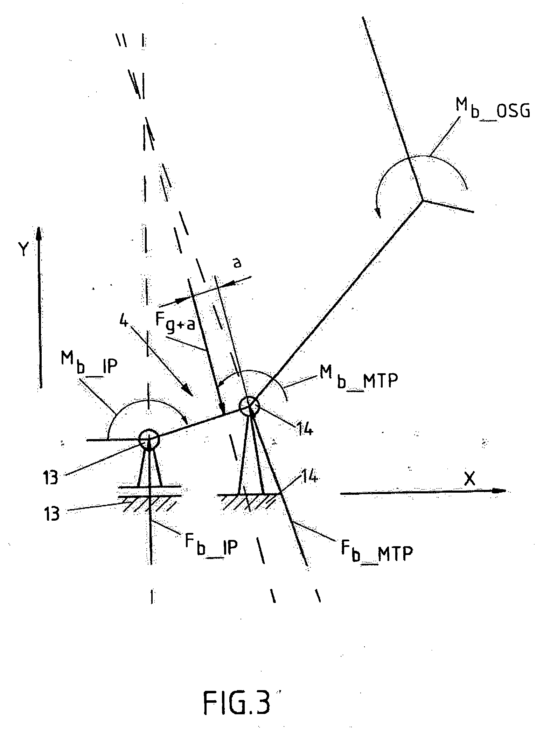

Die Figur 2 zeigt das Fußmodell mit Schuh 2 gemäß Figur 1 mit nunmehr integriert dargestelltem mechanischem Fußmodell 12 während des Gangzyklus der Schwungvorbereitung. Das mechanische Fußmodell 12 stellt ein Hebelmodell dar. Wesentliche Ergänzungen im Vergleich zur Figur 1 ist die Darstellung der Lager 13 und 14 des mechanischen Fußmodells
äDas Lager 13 ist als mechanisches Loslager ausgebildet, das Normalkräfte aufnimmt, und ist gemäß seiner Anordnung dem Großzehenendgelenk zugeordnet. Das Lager 14 ist als mechanisches Festlager ausgebildet und gemäß seiner Anordnung dem Großzehengrundgelenk zugeordnet. In dieser Darstellung greifen die Gewichts- und Beschleunigungskraft des Körperschwerpunktes F_{g+a} und die Bodenreaktionskraft F_{b} am Vorfuß 4 an. Dabei liegen beide Kräfte auf einer Wirkungslinie. Die Figur 3 zeigt nun weiterhin das mechanische Modell während des Gangzyklus der Schwungvorbereitung, wobei zusätzlich zur Beschleunigungskraft des Körperschwerpunktes F_{g+a} die Reaktionskraft am Großzehengrundgelenk F_{b_MTP} und die Reaktionskraft am Großzehenendgelenk F_{b-IP} als Normalkraft eingezeichnet sind. Die Beschleunigungskraft des Körperschwerpunktes F_{g+a} greift weiterhin am Vorfuß 4 an. Die Beschleunigungskraft des Körperschwerpunktes F_{g+a} und die Reaktionskraft am Großzehengrundgelenk F_{b_MTP} liegen nicht auf einer Wirkungslinie, sondern sind um die Strecke a versetzt.

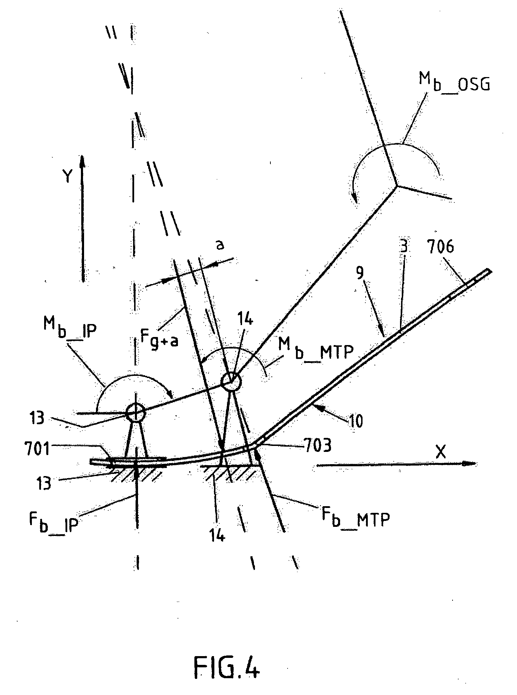

Auch die Figur 4 zeigt weiterhin das mechanische Fußmodell während des Gangzyklus der Schwungvorbereitung, wobei im Unterschied zur Figur 3 eine Schuhsohle 3 eingezeichnet ist, an welcher die Beschleunigungskraft des Körperschwerpunktes F_{g+a} an der Oberseite 9 der Schuhsohle 3 angreift. Ebenso greifen die Reaktionskraft am Großzehengrundgelenk F_{b_MTP} und die Reaktionskraft am Großzehenendgelenk F_{b-IP} an der Unterseite 10 der Schuhsohle 3 an.

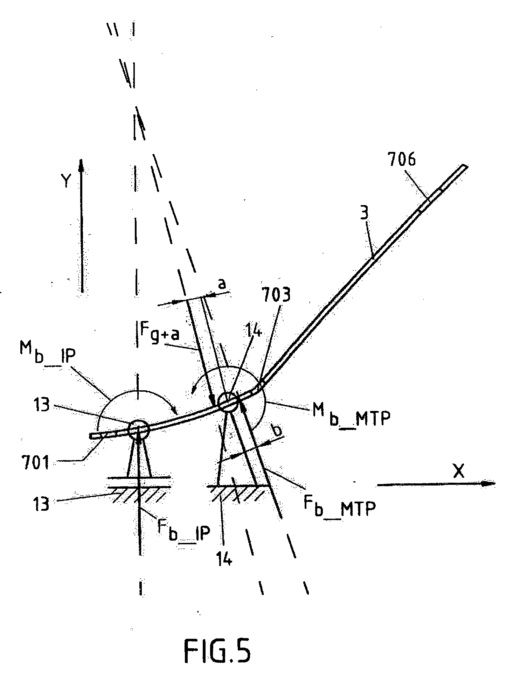

Die Figur 5 zeigt das mechanische Fußmodell mit der Schuhsohle 3 nun ohne Drehmoment am oberen Sprunggelenk zu einem dem Zustand gemäß Figur 4 unmittelbar folgenden Zeitpunkt des Gangzyklus der Schwungvorbereitung. Hier ist aufgrund der erfolgten weitergehenden Fußbewegung der Angriffspunkt der Reaktionskraft am Großzehengrundgelenk F_{b_MTP}um die Strecke b (nach rechts) versetzt.

Mit dem in den Figuren 1-5 dargestellten mechanischen Fußmodell lassen sich erfindungsgemäß die mehraxialen und/oder mehrachsigen Spannungszustände, die an einem menschlichen Fuß bei einer Gehbewegung in einem Schuh 2 auftreten abbilden und mittels der erfindungsgemäßen Vorrichtung als zu ermittelnde Lastgröße(n) erfassen und ermitteln.

Die Figur 6 zeigt schematisch in einer vergrößerten Darstellung ein Ausführungsbeispiel eines Sohlenaufbaus. Ein insgesamt mit 17 bezeichneter Sohlenbereich eines Schuhs 2 umfasst eine Grund- oder Laufsohle 18 und eine in Form einer Einlegsohle schuhinnenseitig darauf angeordnete Schuhsohle 3. Die Grund- oder Laufsohle 18 weist ein nicht näher dargestelltes äußeres Grund- oder Laufprofil 23 auf, das zur Erhöhung der Stabilität beim Laufen beiträgt.

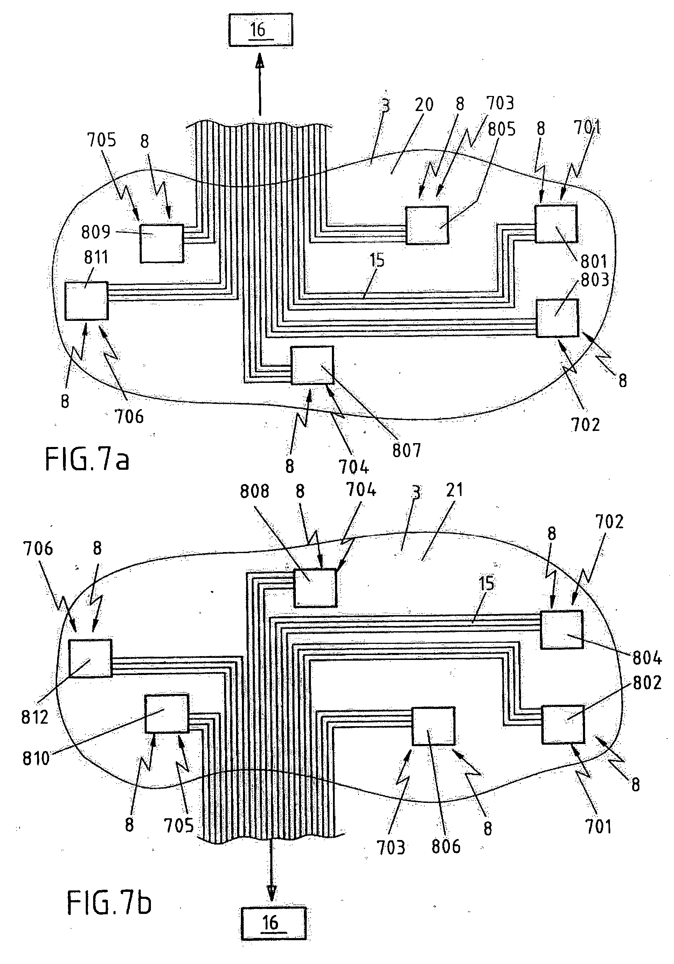

Grundsätzlich ist die erfindungsgemäße Vorrichtung derart ausgebildet, dass eine Messstelle oder ein Messfeld 7 der Trägerschicht 24, insbesondere der Schuhsohle 3, eine erste Oberfläche oder erste Seite und eine zweite Oberfläche oder zweite Seite aufweist, wobei je Messstelle oder je Messfeld jeweils auf der ersten Oberfläche oder Seite und auf der zweiten Oberfläche oder Seite der Trägerschicht 24, insbesondere Schuhsohle 3, gegenüberliegend zueinander angeordnet mindestens ein einen mehrachsigen Spannungs- und/oder Dehnungszustand der Trägerschicht 24, insbesondere Schuhsohle 3, detektierender Kraft- und/oder Dehnungssensor 8 angeordnet ist. Im Ausführungsbeispiel weist die Schuhsohle 3 einen dreischichtigen Aufbau auf. Die mittlere Schicht ist die Trägerschicht 24. Oberseitig, also auf der ersten Seite oder der ersten Oberfläche, befindet sich auf der Trägerschicht 24 eine erste Messschicht 20. In der ersten Messschicht 20 sind an definierten Positionen Messmittel angeordnet und auf der ersten Seite oder der ersten Oberfläche der Trägerschicht 24 kraftschlüssig befestigt, wovon die Messmittel 801 und 802 in der Figur 6 dargestellt sind. Auf der ersten Messschicht 20 ist eine obere elastomere Schicht 19 aufgebracht. Die Schicht 19 dient zum Schutz der Messmittel, im Ausführungsbeispiel also der oberseitig angeordneten Messmittel 801, 803, 805, 807, 809, sowie der (in den Figuren 7a und 7b gezeigten) Mess- und Versorgungsleitungen 15 der Messmittel.

Unterseitig, also auf der zweiten Seite oder der zweiten Oberfläche, ist an der Trägerschicht 24 eine zweite Messschicht 21 angeordnet. Diese zweite Messschicht 21 weist an ebenfalls definierten Positionen, und zwar den oberseitig angeordneten Messmitteln unmittelbar gegenüberliegend, unterseitig angeordnete und auf der zweiten Seite oder der zweiten Oberfläche der Trägerschicht 24 kraftschlüssig befestigte Messmittel 802, 804, 806, 808, 810 und 812 auf. Auf der zweiten Messschicht 21 ist wiederum eine, nun untere, elastomere Schicht 22 aufgebracht. Auch die Schicht 22 dient dem Schutz von Mess- und Versorgungsleitungen 15.

Der die Kraft- und/oder Dehnungssensoren 8 schützend bedeckende Bereich ist dabei aus Silikon hergestellt und der die Trägerschicht 24 bedeckende Bereich ist dabei aus einem flexiblen Ethylenvinylacetat (EVA, 25 - 35 SH) hergestellt.

Die Figuren 7a und 7b zeigen jeweils schematisch eine Draufsicht auf eine Schuhsohle 3, einmal von oben (Fig. 7a) und einmal von unten(Fig. 7b) betrachtet. Die Schuhsohle 3 ist mit den zugehörigen Messmitteln 801 bis 812 versehen. In der vorliegenden bevorzugten Ausführung sind sechs 90°-DMS-Rosetten 801, 803, 805, 807, 809 und 811 in der oberseitigen ersten Messschicht 20 der Schuhsohle 3 angeordnet. Die 90°-DMS-Rosetten 801, 803, 805, 807, 809 und 811 sind in der ersten Messschicht 20 jeweils einem der Messfelder 701 bis 706 eineindeutig zugeordnet. Den 90°-DMS-Rosetten 801, 803, 805, 807, 809 und 811 auf der anderen Seite der Trägerschicht 24 jeweils gegenüberliegend sind in der unterseitigen zweiten Messschicht 21 der Schuhsohle 3 die 90°-DMS-Rosetten 802, 804, 806, 808, 810 und 812 angeordnet. Dabei erfolgt die Zuordnung jeweils paarweise, so dass Kraft- und/oder Dehnungssensoren 8 in Form der 90°-DMS-Rosetten 801 und 802 das Messfeld 701, Kraft- und/oder Dehnungssensoren 8in Form der 90°-DMS-Rosetten 803 und 804 das Messfeld 702, Kraft- und/oder Dehnungssensoren 8 in Form der 90°-DMS-Rosetten 805 und 806 das Messfeld 703, Kraft- und/oder Dehnungssensoren 8 in Form der 90°-DMS-Rosetten 807 und 808 das Messfeld 704, Kraft- und/oder Dehnungssensoren 8 in Form der 90°-DMS-Rosetten 809 und 810 das Messfeld 705 und Kraft- und/oder Dehnungssensoren 8 in Form der 90°-DMS-Rosetten 811 und 812 das Messfeld 706 ausbilden.

Jedes Paar der sich jeweils paarweise gegenüberliegenden 90°-DMS-Rosetten 801 bis 812 ist jeweils mittels einer Wheatstone'schen Messbrücke elektrisch messtechnisch miteinander verbunden und verschaltet. Der Einsatz einer Wheatstone'schen Messbrücke ist hier vorteilhaft, da so auch kleine Veränderungen in den 90°-DMS-Rosetten der Schuhsohle 3 gemessen werden können.

Jede Wheatstone'sche Messbrücke weist vier Leitungen auf, zwei Mess- und zwei Versorgungsleitungen. Die Mess- und Versorgungsleitungen 15 führen zu einer, insbesondere elektronischen, Auswerte- und/oder Datenausleseeinheit 16, die das Auslesen und/oder Verarbeiten der von den Kraft- und/oder Dehnungssensoren 8 in Form der 90°-DMS-Rosetten erzeugten elektrischen Spannungssignale ermöglicht. Insbesondere umfasst diese eine oder mehrere Wheatstone'sche Brücke(n). Das Auslesen oder Übertragen kann kabelgebunden oder leitungsgebunden, aber auch mittels Bluetooth- oder Infrarotschnittstelle und übertragung realisiert werden. Die Ausleseweise mittels Bluetooth ist vorteilhaft, weil sie zum Beispiel auch eine Datenübertragung bei freier Bewegungsmöglichkeit des Probanden ermöglicht. Eine Energieversorgung der elektronischen Auswerte- und/oder Datenausleseeinheit 16 ist beispielsweise mittels einer Batterie möglich.

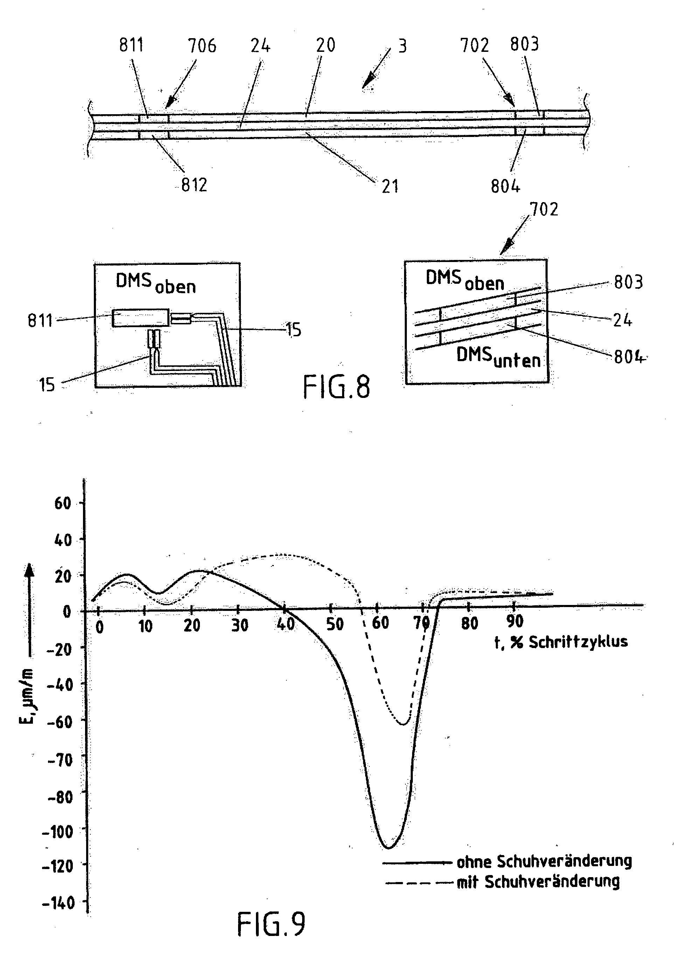

Die Figur 8 zeigt schematisch einen Längsschnitt durch die Schuhsohle 3. Der Schnitt zeigt prinzipiell den schichtartigen Aufbau der Schuhsohle 3. Dargestellt sind die jeweils einander zugeordneten 90°-DMS-Rosetten 803 und 804 des Messfeldes 702 und die 90°-DMS-Rosetten 811 und 812 des Messfeldes 706. Die Trägerschicht 24 befindet sich sandwichartig in der Mitte. Oberseitig ist die Trägerschicht 24 mit der ersten Messschicht 20 und unterseitig ist die Trägerschicht 24 mit der zweiten Messschicht 21 versehen.

Die Schuhsohle 3 weist insgesamt, d.h. inklusive der 90°-DMS-Rosetten, erster Messschicht 20 und zweiter 21 Messschicht sowie der elastomeren Schutz- und Deckschichten 19 und 22, eine Höhe von ca. 3 mm bei einer den deutschen Schuhgrößen von 30 bis 50 entsprechenden Größe der Schuhsohle 3 auf.

Die Trägerschicht 24 besteht aus einer Edelstahlschicht mit einer Dicke von 0,5 mm, wobei die Kraft- und/oder Dehnungssensoren 8 unmittelbar auf deren metallische Oberflächen, insbesondere kraftschlüssig, aufgebracht sind.

In der Figur 8 ist rechts schematisch ein Teilschnitt durch das Messfeld 702 an der Position der 90°-DMS-Rosetten 803 und 804 mit dazwischenliegendem Teilabschnitt der Trägerschicht 24 dargestellt. Diese Darstellung zeigt deutlich die direkt gegenüberliegende Anordnung zweier 90°-DMS-Rosetten 803 und 804.

In der Figur 8 ist links weiterhin schematisch eine Aufsicht auf die 90°-DMS-Rosette 811 mit zugeordneten Mess- und Versorgungsleitungen 15 dargestellt.

Die Figur 9 zeigt mit der erfindungsgemäßen Vorrichtung ermittelte gemittelte Dehnungsverläufe in Längsrichtung der Schuhsohle 3 hinter dem Großzehengrundgelenk während des Gehens, einmal mit (gestrichelte Linie)und einmal ohne (durchgezogene Linie) Schuhveränderung (mittels einer Sohlenrolle).

Zur Aufnahme der Messkurven ging ein Proband (40 Jahre, 70 kg, mit eingeschränkter Großzehenbeweglichkeit) zunächst ohne Schuhveränderung. Dabei wurden die Dehnungen hinter dem Großzehengrundgelenk über 40 Schrittzyklen (1 Schrittzyklus = Bodenkontakt eines Beines bis nächster Bodenkontakt desselben Beines) ermittelt. Die Daten wurden mit einem Analog-DigitalWandler (125 Hz, 16 Bit, 4 Kanäle) gewandelt, per Bluetooth zu einer Auswerteeinheit übertragen und mittels einer LabView basierten Software aufgezeichnet.

Wie in Figur 9 zu sehen ist, stellt sich im Bereich von 30 - 70 % des Schrittzyklus eine deutliche Reduktion der Dehnungen/Verformungen an der Schuhsohle 3 ein. Das Minimum bei ca. 60 % des Schrittzyklus lässt sich durch eine Schuhveränderung in Form einer veränderten Sohlenkonstruktion um 46 % reduzieren. Dies ist das Versorgungsziel der hier gewählten Schuhmodifikation.

Die Figur 9 zeigt weiter, dass die Zielsetzung einer erfolgreichen Ermittlung der Vorfußbeanspruchung mittels der erfindungsgemäßen Vorrichtung und des erfindungsgemäßen Verfahrens realisiert werden kann, weil eine systematische Ermittlung der auftretenden Dehnungen/Verformungen/Spannungen, d.h. der auftretenden mehraxialen Spannungszustände, mit der erfindungsgemäß ausgestatteten Schuhsohle 3 während des Gehens möglich und umsetzbar ist.

Ferner können die erfindungsgemäße Vorrichtung und das erfindungsgemäße Verfahren erfolgreich bei einer Überprüfung von orthopädisch-technischen Maßnahmen für Diabetikerschuhe eingesetzt werden. In der praktischen Umsetzung muss hierfür unter anderem der betroffene Fuß mittels orthopädischer Hilfsmittel komplett entlastet werden. Zum Einsatz kommen Versteifungen des gesamten Schuhs und sogenannte Sohlenrollen. Die Versorgungskontrollen werden bisher mit Druckmesssohlen nach dem Stand der Technik durchgeführt, was aber nur unzureichende Ergebnisse liefert, weil damit nur Normaldrücke gemessen werden können. Erfindungsgemäß kann mit einer vorstehend beschriebenen Schuhsohle 3 und der dazugehörigen Messtechnik die Wirksamkeit von Schuhveränderungen deutlich besser und gezielter überprüft werden, weil geringere mehraxiale Verformungen der Schuhsohle 3 kleinere Dehnungen und somit kleinere Spannungen und Momente bedeuten, die mittels der Erfindung aber erfassbar sind. Auf diesem Weg lässt sich auf eine Reduktion der Fußbeanspruchung schließen.

Ähnliche Messungen und Schlussfolgerungen sind auch auf dem Gebiet der Patientenversorgung mit Prothesen realisierbar. Prothesen dienen dem Ersatz amputierter Gliedmaßen. Sie sollen einen guten Funktionsersatz an der betroffenen Stelle gewährleisten. Bezogen auf die Beine bedeutet dies, dass gut angepasste Prothesen Stabilität und Sicherheit gewährleisten und die Mobilität der Patienten wiederherstellen sollen.

Da der Vorfußhebel ein entscheidender Faktor beim Gehen ist, wird auch mit Prothesenversorgungen versucht, eine möglichst große Hebelwirkung zu erzielen. Die von Prothesenfüßen übertragenen Momente können mit der erfindungsgemäßen Vorrichtung und dem erfindungsgemäßen Verfahren ebenfalls überprüft werden. Gleichfalls ist ein Vergleich zwischen der erhaltenen und der amputierten Seite möglich.

Bei großen und lang andauernden Beanspruchungen der Füße, wie sie beim Marathonlauf auftreten, kommt es bei Läufern häufig zu Stressfrakturen oder Überbelastungsfrakturen im Mittel- und Vorfußbereich. Die Ursachen dieser Traumata, aber auch die auftretenden mehrachsigen Belastungen während des Laufens, sind noch unbekannt. Mit einer Schuhsohle 3 ist eine Beanspruchungsanalyse während des Laufens möglich. Besonders vorteilhaft ist in diesem Zusammenhang das Auslesen der Messdaten mittels Bluetooth, weil dies eine Übertragung der Messdaten bzw. der erzeugten elektrischen (Spannungs)Signale auch während des Laufens und somit eine kurzfristige mehraxiale Spannungsanalyse ermöglicht.

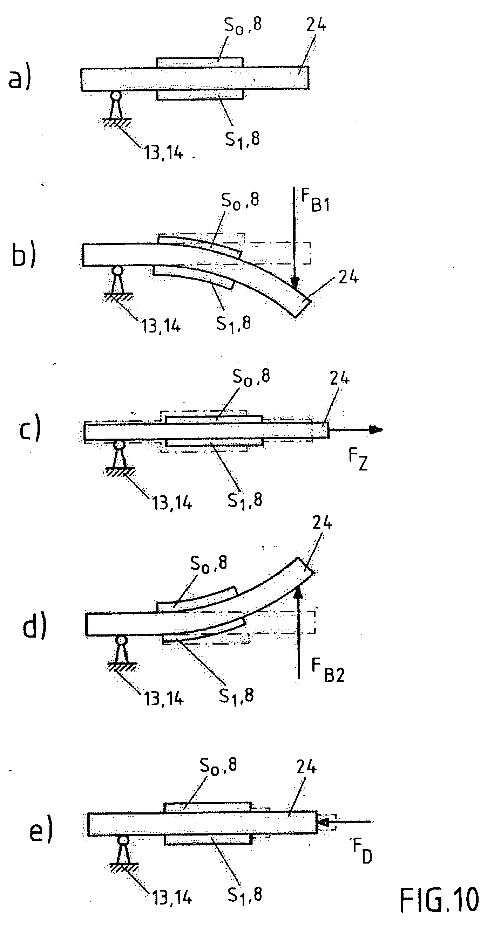

Biegungen und/oder Torsionen und dabei auftretende Momente sind durch einzelne Sensoren nicht direkt messbar.
Im Unterschied zu beispielsweise gleichmäßig auf einer Fläche verteilten Normalbeanspruchungen, die durch reine Zug- oder Druckkräfte erzeugt werden, treten bei Biegungen oder Torsionen zudem sogenannte Spannungsverläufe auf. Auf einer Seite der Trägerschicht 24 oder der Schuhsohle 3 oder Messsohle kommt es zu positiven und auf der gegenüberliegenden Seite zu negativen Verformungen. Diese werden bei dem vorstehend beschriebenen System mittels der erfindungsgemäßen Vorrichtung und dem erfindungsgemäßen Verfahren indirekt über Kraft- und/oder Dehnungssensoren S₀ und S₁ bzw. 8 an der dazwischenliegenden Trägerschicht in den einzelnen Messfeldern erfasst, wie dies Fig. 10a-10e zeigt. Dargestellt ist jeweils eines der Messfelder 701-706.
Wie wichtig die gegenüberliegende Anordnung der Messmittel 8 (Sensoren, S₀ und S_{1 bzw}. ₈) für die richtige, trennscharfe Ermittlung der auftretenden Belastungen und Beanspruchung ist, wird aus Fig. 10 deutlich.

Eine Differenzierung von Zugbelastungen (Fig. 10c) und Biegebelastungen (Fig. 10b) ist nur aufgrund der oberseitigen und unterseitigen Anordnung der Druck- und/oder Messsensoren 8 bzw. S₀ und S₁ an der Trägerschicht 24 möglich. Tritt eine Biegung an der jeweiligen Messstelle auf, kommt es zu einer positiven Verformung des Sensors S₀ und einer negativen des Sensors S₁ (Fig. 10b). Bei Zug werden hingegen beide Sensoren S₀ und S₁ positiv verformt (Fig. 10c). Die Trägerschicht 24 oder die Sohle 3 besitzt im Messfeld in Längs- und Querrichtung einen spiegelsymmetrischen Querschnitt. Daraus ergibt sich, dass die bei Biegung ermittelten positiven und negativen Verformungen den gleichen Betrag, aber entgegengesetzte Vorzeichen, besitzen. Dies ermöglicht die durch spezielle elektrische Verschaltungen (Wheatstone-Brückenschaltungen) gegebenen Möglichkeiten der Addition von Messsignalen und der Kompensation von Störgrößen. Befindet sich auf oder in der Sohle nur ein Sensor, z.B. So, so würde dieser bei jeder der beiden Beanspruchungsarten Zug und Biegung das gleiche Ergebnis liefern, da der Sensor S₀ in beiden Fällen positiv gedehnt wird. Daraus ergibt sich, dass durch den Einsatz eines Sensors keine Unterscheidung und Interpretation der Zug- und Biegebelastung möglich ist. Gleiches gilt für die Abgrenzung von einer Druckbeanspruchung (Fig. 10e) gegenüber einer Biegebeanspruchung (Fig. 10d) und bei der Ermittlung von Torsionsbelastungen. In gepunkteten Linien ist in den Figuren 10b-10e jeweils der unbelastete Ausgangszustand gemäß Fig. 10a dargestellt.

## Patentansprüche

1. Vorrichtung zur Erfassung mindestens einer an einem Teil eines biologischen Systems auftretenden mechanischen Lastgröße, wobei die Vorrichtung eine an dem Teil, insbesondere einer Extremität, einem Gelenk oder einem Knochen, des biologischen Systems lastaufnehmend angeordnete oder anordnenbare, elastisch verformbare Trägerschicht (24) aufweist, an welcher mindestens ein eine Messstelle oder ein Messfeld (7) ausbildendes Messmittel angeordnet ist, das mindestens einen Kraft- und/oder Dehnungssensor (8) aufweist,
**dadurch gekennzeichnet,**
**dass** die mindestens eine Messstelle oder das mindestens eine Messfeld (7) mindestens zwei an der Trägerschicht (24) einander gegenüberliegend angeordnete Messmittel, insbesondere Kraft- und/oder Dehnungssensoren (8), aufweist.

2. Vorrichtung nach Anspruch 1, **dadurch gekennzeichnet, dass** das Teil ein menschlicher Fuß ist.

3. Vorrichtung nach Anspruch 1 oder 2, **dadurch gekennzeichnet, dass** die Trägerschicht (24) Bestandteil einer Schuhsohle (3) ist oder eine solche ausbildet oder dass die Trägerschicht (24) Bestandteil einer Schuhsohle (3) ist oder eine solche ausbildet, wobei die Schuhsohle (3) als herausnehmbare Einlegesohle ausgebildet ist.

4. Vorrichtung nach einem der vorhergehenden Ansprüche, **dadurch gekennzeichnet, dass** mindestens eines der Messmittel ein einen mehrachsigen Spannungs- und/oder Dehnungszustand, insbesondere eine elastische Verformung, der Trägerschicht (24), insbesondere Schuhsohle (3), detektierender Kraft- und/oder Dehnungssensor (8) ist.

5. Vorrichtung nach einem der vorhergehenden Ansprüche,
**dadurch gekennzeichnet, dass** mindestens einer der Kraft- und/oder Dehnungssensoren (8) ein einen statischen und/oder dynamischen mehrachsigen Spannungs- und/oder Dehnungszustand der Trägerschicht (24), insbesondere Schuhsohle (3), detektierender Kraft- und/oder Dehnungssensor (8) ist.

6. Vorrichtung nach einem der vorhergehenden Ansprüche, **dadurch gekennzeichnet, dass** mindestens einer der Kraft- und/oder Dehnungssensoren (8) ein Biegemomente und/oder Torsionsmomente und/oder Biegespannungen und/oder Torsionsspannungen der Trägerschicht (24), insbesondere Schuhsohle (3), detektierender Kraft- und/oder Dehnungssensor (8) ist.

7. Vorrichtung nach einem der vorhergehenden Ansprüche, **dadurch gekennzeichnet, dass** die jeweils mindestens zwei an einer Messstelle oder einem Messfeld (7) einander gegenüberliegend angeordneten Messmittel, insbesondere Kraft- und/oder Dehnungssensoren (8), einen mehrachsigen Spannungs- und/oder Dehnungszustand, insbesondere eine elastische Verformung, der Trägerschicht (24), insbesondere Schuhsohle (3), detektierende Kraft- und/oder Dehnungssensoren (8) sind.

8. Vorrichtung nach einem der vorhergehenden Ansprüche, **dadurch gekennzeichnet, dass** eine Messstelle oder ein Messfeld (7) eine Trägerschicht (24), insbesondere Schuhsohle (3), mit einer ersten Oberfläche und einer zweiten Oberfläche aufweist, wobei je Messstelle oder je Messfeld (7) jeweils auf der ersten und auf der zweiten Oberfläche der Trägerschicht (24), insbesondere Schuhsohle (3), mindestens ein einen mehrachsigen Spannungs- und/oder Dehnungszustand der Trägerschicht (24), insbesondere Schuhsohle (3), detektierter Kraft- und/oder Dehnungssensor (8) angeordnet ist.

9. Vorrichtung nach einem der vorhergehenden Ansprüche, **dadurch gekennzeichnet, dass** die Schuhsohle (3) oder die Trägerschicht (24) aus einem Material, insbesondere Edelstahl, besteht, das bei einer natürlichen Belastung durch einen menschlichen Fuß ausschließlich in seinem elastischen Bereich beansprucht, insbesondere verformt, wird.

10. Vorrichtung nach einem der vorhergehenden Ansprüche, **dadurch gekennzeichnet, dass** der mindestens eine Kraft- und/oder Dehnungssensor (8), vorzugsweise alle Kraft- und/oder Dehnungssensoren (8), als Dehnungsmessstreifen (DMS) ausgebildet ist/sind.

11. Vorrichtung nach einem der vorhergehenden Ansprüche, **dadurch gekennzeichnet, dass** sie einen Schuh (2) umfasst, der eine Grund- oder Laufsohle (18) und eine auf der Grund- oder Laufsohle (18) angeordnete Schuhsohle (3), die die Messstellen oder die Messfelder (7) aufweist, umfasst.

12. Vorrichtung nach einem der vorhergehenden Ansprüche, **dadurch gekennzeichnet, dass** sie insbesondere die Schuhsohle (3), eine erste elastomere Schutzschicht (19) und eine darunter angeordnete erste Messschicht (20) auf einer ersten Seite der Trägerschicht (24) sowie eine auf einer zweiten Seite der Trägerschicht (24)angeordnete zweite Messschicht (21) und eine die zweite Messschicht (21) schützend überdeckende zweite elastomere Schutzschicht (22) aufweist.

13. Verfahren zur Erfassung mindestens einer an einem Teil, insbesondere einer Extremität, einem Gelenk oder einem Knochen, eines biologischen Systems auftretenden mechanischen Lastgröße, wobei an dem Teil eine elastisch verformbare Trägerschicht (24) lastaufnehmend angeordnet wird und wobei die Lastgröße an der elastisch verformbaren Trägerschicht (24) mittels mindestens eines dort angebrachten Messmittels, insbesondere mittels mindestens eines Kraft- und/oder Dehnungssensors (8), ermittelt wird, **dadurch gekennzeichnet, dass** die mechanische Lastgröße mittels mindestens zweier, an einer Messstelle oder einem Messfeld (7) einander an der Trägerschicht (24) gegenüberliegend angeordneter Messmittel, insbesondere Kraft- und/oder Dehnungssensoren (8), erfasst wird.

14. Verfahren nach Anspruch 13, **dadurch gekennzeichnet, dass** als mechanische Lastgröße ein mehrachsiger Spannungs- und/oder Dehnungszustand, insbesondere eine elastische Verformung, der Trägerschicht (24) erfasst wird.

15. Verfahren nach Anspruch 13 oder 14, **dadurch gekennzeichnet, dass** die Trägerschicht (24) oder die Schuhsohle (3) bei ihrer lastaufnehmenden Belastung durch das Teil des biologischen Systems ausschließlich im elastischen Bereich des die Trägerschicht (24) oder die Schuhsohle (3) ausbildenden Materials beansprucht, insbesondere verformt, wird.

16. Verwendung einer Vorrichtung nach einem der Ansprüche 1-12 zur Durchführung eines Verfahrens nach einem der Ansprüche 13-15.

## Claims

1. Device for recording at least one mechanical load magnitude occurring at a part of a biological system, wherein the device comprises an elastically deformable substrate layer (24) arranged or arrangeable to absorb a load on the part, in particular an extremity, a joint or a bone, of the biological system, at which at least one measuring instrument forming a measuring point or a measuring field (7) is arranged, which comprises at least one force and/or expansion sensor (8),
**characterised in that**,
the at least one measuring point or the at least one measuring field (7) comprises at least two opposingly arranged measuring instruments, in particular force and/or expansion sensors (8), on the substrate layer (24).

2. Device according to claim 1, **characterised in that** the part is a human foot.

3. Device according to claim 1 or 2, **characterised in that** the substrate layer (24) is part of a shoe sole (3) or forms such a one, or **in that** the substrate layer (24) is part of a shoe sole (3) or forms such a one, wherein the shoe sole (3) is designed as a removable insole.

4. Device according to one of the preceding claims, **characterised in that** at least one of the measuring instruments is a force and/or expansion sensor (8) detecting a multi-axis tensioning and/or expansion condition, in particular an elastic deformation of the substrate layer (24) in particular a shoe sole (3).

5. Device according to one of the preceding claims, **characterised in that** at least one of the force and/or expansion sensors (8) is a force and/or expansion sensor (8) detecting a static and/or dynamic multi-axis tensioning and/or expansion condition of the substrate layer (24), in particular a shoe sole (3).

6. Device according to one of the preceding claims, **characterised in that** at least one of the force and/or expansion sensors (8) is a force and/or expansion sensor (8) detecting a bending moment and/or torsion moment and/or bending tension and/or torsion of the substrate layer (24), in particular a shoe sole (3).

7. Device according to one of the preceding claims, **characterised in that** the respective at least two oppositely arranged measuring instruments, in particular force and/or expansion sensors (8), are force and/or expansion sensors (8) detecting a multi-axis tensioning and/or expansion condition, in particular an elastic deformation, of the substrate layer (24), in particular a shoe sole (3), at a measuring point or a measuring field (7).

8. Device according to one of the preceding claims, **characterised in that** a measuring point or a measuring field (7) comprises a substrate layer (24), in particular a shoe sole (3), with a first surface and a second surface, wherein at least one force and/or expansion sensor (8) detecting a multi-axis tensioning and/or expansion condition of the substrate layer (24), in particular a shoe sole (3) is arranged per measuring point or per measuring field (7), each on the first and on the second surface of the substrate layer (24), in particular a shoe sole (3).

9. Device according to one of the preceding claims, **characterised in that** the shoe sole (3) or the substrate layer (24) consists of a material, in particular stainless steel, that is subjected to stress during a natural load application through a human foot, in particular deformed, only in its elastic area.

10. Device according to one of the preceding claims, **characterised in that** the at least one force and/or expansion sensor (8), preferably all force and/or expansion sensors (8), is/are designed as strain gauge strips (SGS).

11. Device according to one of the preceding claims, **characterised in that** it comprises a shoe (2) that comprises a base or running sole (18) and a shoe sole (3) arranged on the base or running sole (18), which comprises the measuring points or the measuring fields (7).

12. Device according to one of the preceding claims, **characterised in that** it in particular comprises the shoe sole (3), a first elastomeric protection layer (19) and a first measuring layer (20) arranged under the same, on a first side of the substrate layer (24), as well as a second measuring layer (21) arranged on a second side of the substrate layer (24), and a second elastomeric protection layer (22) protectively covering the second measuring layer (21).

13. Method for recording at least one mechanical load magnitude occurring at a part, in particular an extremity, a joint or a bone, of the biological system, wherein an elastically deformable substrate layer (24) is arranged to absorb a load on the part, and wherein the load magnitude on the elastically deformable substrate layer (24) is determined by means of at least one measuring instrument positioned there, in particular by means of at least one force and/or expansion sensor (8), **characterised in that** the mechanical load magnitude is recorded by means of at least two measuring instruments, in particular force and/or expansion force sensors (8), opposingly arranged at a measuring point or a measuring field (7) on the substrate layer (24).

14. Method according to claim 13, **characterised in that** a multi-axis tensioning and/or expansion condition, in particular an elastic deformation, of the substrate layer (24) is recorded as a mechanical load magnitude.

15. Method according to claim 13 or 14, **characterised in that** the substrate layer (24) or the shoe sole (3) is subjected to stress, in particular deformed, during a load absorbing application by the part of the biological system only in the elastic area of the material forming the substrate layer (24) or the shoe sole (3).

16. Use of a device according to one of the claims 1-12 for carrying out a method according to one of the claims 13-15.

## Revendications

1. Dispositif pour déterminer au moins une valeur de la charge mécanique apparaissant sur une partie d'un système biologique, le dispositif comportant une couche support (24), pouvant subir une déformation élastique, disposée ou pouvant être disposée, avec possibilité de supporter la charge, contre la partie, en particulier une extrémité, une articulation ou un os, du système biologique, couche contre laquelle est disposé au moins un moyen de mesure configuré comme un point de mesure ou un champ de mesure (7), moyen qui comprend au moins un capteur de force et/ou de déformation (8), **caractérisé en ce que** les points de mesure ou le ou les champs de mesure (7) comprennent au moins deux moyens de mesure, disposés opposés l'un à l'autre contre la couche support (24), en particulier des capteurs de force et/ou de déformation (8).

2. Dispositif selon la revendication 1, **caractérisé en ce que** la partie est un pied humain.

3. Dispositif selon la revendication 1 ou 2, **caractérisé en ce que** la couche support (24) est un constituant d'une semelle de chaussure (3) ou forme une telle semelle, ou que la couche support (24) est un constituant d'une semelle de chaussure (3) ou forme une telle semelle, la semelle de chaussure (3) étant configurée comme une semelle amovible à insérer.

4. Dispositif selon l'une des revendications précédentes, **caractérisé en ce qu'**au moins l'un des moyens de mesure est un capteur de force et/ou de déformation (8), qui détecte un état de contrainte et/ou de déformation multiaxe, en particulier une déformation élastique, de la couche support (24), en particulier de la semelle de chaussure (3).

5. Dispositif selon l'une des revendications précédentes, **caractérisé en ce qu'**au moins l'un des capteurs de force et/ou de déformation (8) est un capteur de force ou de déformation (8) détectant un état de contrainte et/ou de déformation statique et/ou dynamique multiaxe de la couche support (24), en particulier de la semelle de chaussure (3).

6. Dispositif selon l'une des revendications précédentes, **caractérisé en ce qu'**au moins l'un des capteurs de force et/ou de déformation (8) est un capteur de force et/ou de déformation (8) qui détecte des moments de flexion et/ou des moments de torsion et/ou des contraintes de flexion et/ou des contraintes de torsion de la couche support (24), en particulier de la semelle de chaussure (3).

7. Dispositif selon l'une des revendications précédentes, **caractérisé en ce que** chaque groupe d'au moins deux moyens de mesure disposés opposés l'un à l'autre contre un point de mesure ou un champ de mesure (7), en particulier des capteurs de force et/ou de déformation (8), sont des capteurs de force et/ou de déformation (8) qui détectent un état de contrainte et/ou de déformation multiaxe, en particulier une déformation élastique, de la couche support (24), en particulier de la semelle de chaussure (3).

8. Dispositif selon l'une des revendications précédentes, **caractérisé en ce qu'**un point de mesure ou un champ de mesure (7) comprend une couche support (24), en particulier la semelle de chaussure (3), avec une première surface et une deuxième surface, au moins un capteur de force et/ou de déformation (8) qui détecte un état de contrainte et/ou de déformation multiaxe de la couche support (24), en particulier de la semelle de chaussure (3), étant disposé pour chaque point de mesure ou chaque champ de mesure (7), dans chaque cas sur la première et sur la deuxième surfaces de la couche support (24), en particulier de la semelle de chaussure (3).

9. Dispositif selon l'une des revendications précédentes, **caractérisé en ce que** la semelle de chaussure (3) ou la couche support (24) est constituée d'un matériau, en particulier l'acier inoxydable, qui dans le cas d'une contrainte naturelle sous l'effet d'un pied humain est sollicité exclusivement dans sa zone élastique, en particulier par déformation.

10. Dispositif selon l'une des revendications précédentes, **caractérisé en ce que** le ou les capteurs de force et/ou de déformation (8), de préférence tous les capteurs de force et/ou de déformation (8), sont conçus comme des jauges extensométriques (DMS).

11. Dispositif selon l'une des revendications précédentes, **caractérisé en ce qu'**il comprend une chaussure (2), qui comprend une semelle de base ou une semelle de marche (18) et une semelle de chaussure (3) disposée sur la semelle de base ou la semelle de marche (18), qui comprend les points de mesure ou les champs de mesure (7).

12. Dispositif selon l'une des revendications précédentes, **caractérisé en ce qu'**il comprend en particulier la semelle de chaussure (3), une première couche de protection élastomère (19) et une première couche de mesure (20), disposée en-dessous, sur la première face de la couche support (24), ainsi qu'une deuxième couche de mesure (21), disposée sur une deuxième face de la couche support (24), et une deuxième couche de protection élastomère (22), qui recouvre avec effet protecteur la deuxième couche de mesure (21).

13. Procédé pour déterminer au moins une valeur de la charge mécanique apparaissant sur une partie, en particulier une extrémité, une articulation ou un os, d'un système biologique, dans lequel une couche support (24) pouvant subir une déformation élastique est disposée avec possibilité de supporter la charge contre la partie, la valeur de la charge contre la couche support (24) pouvant subir une déformation élastique étant déterminée à l'aide d'au moins un moyen de mesure qui y est rapporté, en particulier à l'aide d'au moins un capteur de force et/ou de déformation (8), **caractérisé en ce que** la valeur de la charge mécanique est déterminée à l'aide d'au moins deux moyens de mesure, en particulier des capteurs de force et/ou de déformation (8), disposés contre un point de mesure ou un champ de mesure (7), opposés l'un à l'autre contre la couche support (24).

14. Procédé selon la revendication 13, **caractérisé en ce qu'**on détermine en tant que valeur de la charge mécanique un état de contrainte et/ou de déformation multiaxe, en particulier une déformation, de la couche support (24).

15. Procédé selon la revendication 13 ou 14, **caractérisé en ce que** la couche support (24) ou la semelle de chaussure (3) subit, quand elle est soumise sous l'effet de la partie du système biologique à une contrainte avec possibilité de supporter la charge, une sollicitation, en particulier une déformation, exclusivement dans la zone élastique du matériau formant la couche support (24) ou la semelle de chaussure (3).

16. Utilisation d'un dispositif selon l'une des revendications 1 à 12 pour la mise en oeuvre d'un procédé selon l'une des revendications 13 à 15.
